# EUROPEAN PATENT SPECIFICATION

(11) **EP 2 119 452 B1**
(45) Date of publication and mention of the grant of the patent: **04.03.2020**
(21) Application number: 07855983.8
(22) Date of filing: 24.12.2007
(51) Int. Cl.: A61K 39/00, C07K 16/28

(54) **PHARMACEUTICAL COMPOSITION, COMPRISING AN ANTI-CD6 MONOCLONAL ANTIBODY USED IN THE DIAGNOSIS AND TREATMENT OF RHEUMATOID ARTHRITIS**
PHARMAZEUTISCHE ZUSAMMENSETZUNG MIT EINEM ANTI-CD6-MONOKLONALEN ANTIKÖRPER BEI DER DIAGNOSE UND BEHANDLUNG VON RHEUMATOIDER ARTHRITIS
COMPOSITION PHARMACEUTIQUE CONTENANT UN ANTICORPS MONOCLONAL ANTI-CD6 UTILISE DANS LE DIAGNOSTIC ET LE TRAITEMENT DE L'ARTHRITE RHUMATOIDE

(30) Priority: 26.12.2006 CU 20060250
(43) Date of publication of application: 18.11.2009
(60) Divisional application: 19169359.7
(73) Proprietor: Centro de Inmunologia Molecular, Ciudad de la Habana 12100 (CU)
(72) Inventor: MONTERO CASIMIRO, José Enrique, Ciudad De La Habana 17100 (CU); CASACO PARADA, Ángel Raimundo, Ciudad de la Habana (CU); MAZORRA HERRERA, Zaima, Ciudad De La Habana (CU); ALONSO RAMIREZ, Ruby, San José de las Lajas, Provincia Habana (CU); PEREZ RODRIGUEZ, Rolando, Ciudad De La Habana (CU)
(74) Representative: Schüssler, Andrea
(86) International application number: PCT/CU2007/000021
(87) International publication number: WO 2008/077355

(56) References cited:
- EP-A2- 0 807 125
- WO-A1-00/67796
- WO-A1-95/12614
- WO-A1-98/43089
- WO-A2-97/19111
- ES-A1- 2 192 128
- ES-T3- 2 254 174
- US-B1- 6 372 215
- US-B1- 6 572 857
- JOO YEONG-SHIL ET AL: "Evidence for the expression of a second CD6 ligand by synovial fibroblasts", ARTHRITIS AND RHEUMATISM, vol. 43, no. 2, February 2000 (2000-02), pages 329-335, XP002669703, ISSN: 0004-3591
- E Montero ET AL: Arthritis Research, vol. 4, no. Suppl 1, 1 January 2002 (2002-01-01), page 114, XP055118708, ISSN: 1465-9905, DOI: 10.1186/ar450

## Description

### Field of the Invention.

The present invention is related to the human medicine and especially with the generation of pharmaceutical compositions comprising a humanized monoclonal antibody recognizing the leukocyte differentiation antigen CD6, and particularly with therapeutic formulations which contain a humanized monoclonal antibody that recognizes the leukocyte differentiation antigen CD6 able to induce a long-lasting therapeutic effect in Rheumatoid Arthritis patients.

### Description of the Prior Art.

Autoimmune Diseases and particularly, Rheumatoid Arthritis **(RA)** have available different strategies with therapeutic purposes, associated to their known immunopathologic mechanisms (Smolen, J.S. et al. (2003) Nature Reviews Drug Discovery 2:473; Feldmann, M. et al. (2005) Nature 435:612). However, there is not a treatment for Rheumatoid Arthritis that administered for a short period of time may induce a long-lasting clinical response (Garber, K. (2005) Nature Biotechnology 23(11):1323).

Several biotherapies are currently available for the treatment of patients with Autoimmune Diseases and particularly for therapy of Rheumatoid Arthritis patients, although their clinical effect as monotherapies is very limited (Olsen, N.J. et al. (2004) N Engl J Med 350:2167). Moreover, combinatorial therapies of biological agents with Methotrexate may induce clinical remission in 30 - 40 % of Rheumatoid Arthritis patients, but the disease remains significantly active in most of the patients despite the treatment and complete remissions rarely occur (Pincus, T. et al. (1999) Ann Intern Med 131:768; Olsen, N.J. et al. (2004) N Engl J Med 350:2167). Additionally, multiple and severe adverse events have been described for such therapeutic approximations (O'Dell, J.R. (2004) N Engl J Med 350:2591).

Monoclonal antibodies **(mAbs)** are biological agents used in the treatment of Autoimmune Diseases (Feldmann, M. et al. (2005) Nature 435:612). Particularly, monoclonal antibodies depleting with the capacity to deplete autoreactive cells are of medical interest due to the consideration that a prolong depletion of lymphocytes is required to induce a therapeutic effect in Autoimmune Disease patients, such as Rheumatoid Arthritis (Edwards, J.C. et al. (2004) N Engl J Med 350:2572; Stohl, W. et al. (2006) Clinical Immunology 121:1; Browning, J.L. (2006) Nature Reviews Drug Discovery 5:564). This immunosuppression induced by lymphocyte depletion is a common mechanism of therapeutic effect for several therapeutic options in Autoimmune Diseases (Kahan, B.D. (2003) Nature Reviews Immunology 3:831). Nevertheless, therapeutic effects are limited and associated to significant adverse events (Edwards, J.C. et al. (2004) N Engl J Med 350:2572; Hale, D.A. (2004) Current Opinion Immunology 16:565; Goldblatt, F. et al. (2005) Clinical and Experimental Immunology 140:195).

Consequently, a better understanding of molecules and mechanisms involved in the physiopathology of Autoimmune Diseases, and particularly in Rheumatoid Arthritis, should lead to the discovery of new therapeutic targets where the intended therapy switch from the elimination of autoreactive cells to the induction or the restoration of the mechanism of tolerance and immunoregulation in the patient (Taylor, P.C. et al. (2004) Current Opinion Pharmacology 4:368; Feldmann, M. et al. (2005) Nature 435:612). The leukocyte differentiation antigen CD6 is a molecule no well studied and characterized yet. The CD6 is a surface glycoprotein expressed primarily in T lymphocytes and in a minor subpopulation of B lymphocytes in peripheral blood of normal individuals. However the **origin** and the functional characterization in these cells **are** very limited. Basically, it is considered that CD6 in these cells is a receptor with co-stimulatory function, but the mechanism in unknown (Aruffo, A. et al. (1997) Immunol Today 18(10):498; Patel, D.D. (2000) J Biol Regul Homeost Agents 2000 14(3):234). Its expression in mature thymocytes has been associated to the lymphocyte maturation process in this lymphoid organ (Singer, N.G. et al. (2002) Int Immunol. 14(6):585).

The CD6 model has been of therapeutic interest and it has been claimed that the mechanism of action for anti-CD6 monoclonal antibodies is based in their capability to inhibit and modulate the CD6 binding to its ligand named ALCAM (Activated Leukocyte Adhesion Molecule) (US Patent 6372215). Consequently, those monoclonal antibodies are considered useful for the treatment of Autoimmune Diseases, but therapeutic evidences in patients based on such claim are not substantially documented.

CD6 molecule is recognized by the mouse monoclonal antibody ior-t1. Therapeutic formulations of this murine monoclonal antibody have therapeutic effect in Psoriasis (Montero, E. et al. (1999) Autoimmunity 29(2):155). Subsequently, based on methods of genetic engineering (Patent No.0699755 E.P. Bul.) it was obtained a humanized version of this mouse anti-human CD6 monoclonal antibody designated T1h (EP 0 807 125 A2).

The novelty of this invention relies on the generation of therapeutic formulations containing anti-CD6 monoclonal antibodies for their use in Autoimmune Disease patients and particularly, in Rheumatoid Arthritis patients. Surprisingly, the administration of the humanized T1h monoclonal antibody during 6 weeks as a monotherapy, in Autoimmune Disease patients and particularly in Rheumatoid Arthritis patients with a prolonged evolution and in active phase of the disease, induce a long-lasting therapeutic effect lasting for months after finished the administration of the treatment, without significant adverse events. This effect is not associated to the depletion of the CD6+ cells because the recovery to the normal value of that cellular subpopulation does not impede a persistent clinical improvement of the disease. The humanized T1h monoclonal antibody does not inhibit the binding of CD6 to the ALCAM, effect that was previously claimed for therapies in this therapeutic model, indicating its potential association to an alternative mechanism. Additionally, T1h treatment may sensitize autoimmune cells to the effect of anti-inflammatory agents, steroids or chemotherapies as Methotrexate, which may lead to the combinatorial use of humanized monoclonal antibody T1h with other drugs or other biotherapies.

### Detailed description of the Invention.

The present invention relates with therapeutic formulations of monoclonal antibodies that recognize the human antigen CD6, effective in the treatment of patients with Autoimmune Diseases. More particularly, the present invention comprises the use of pharmaceutical compositions containing the humanized monoclonal antibody T1h, which recognizes the human leukocyte differentiation antigen CD6 and their use for the diagnosis and treatment of the Rheumatoid Arthritis.

It is a subject of the present invention a therapeutic formulation containing the humanized monoclonal antibody T1h, which induces a long-lasting therapeutic effect in Rheumatoid Arthritis patients by the recognition of the CD6 molecule. The term "humanized Monoclonal Antibody" refers to a monoclonal antibody obtained by genetic engineering methods as described in the patent No. 0699755 (E.P. Bul).

### 1. - Generation of pharmaceutical compositions contains the humanized anti-human CD6 Monoclonal Antibody T1h.

The pharmaceutical composition of the present invention contains a humanized monoclonal antibody T1h that recognizes the human leukocyte differentiation antigen CD6. The humanized **anti-human** CD6 monoclonal antibody T1h is obtained from the secreting hybridoma IOR- T1A with deposit No. ECACC 96112640, as described in (EP 0 807 125 A2). Additionally, this composition contains as an appropriate excipient a physiological buffered solution, similar to others used for the formulation of monoclonal antibodies for intravenous use, as described in EP 0807125. The composition of the present invention is administered in the way of injections in a range of doses from 0.05 to 1 mg/Kg of body weight

### 2.- Characterization of the therapeutic effect of the pharmaceutical compositions containing the humanized anti-human CD6 Monoclonal Antibody T1h.

Rheumatoid Arthritis patients diagnosed according to the standard criteria and in an active phase of the disease, resistant to conventional therapies, such as anti-inflammatory drugs, steroids or chemotherapeutic agents (e.g.: Methotrexate) are susceptible to the administration of the humanized monoclonal antibody T1h as monotherapy or in combination with anti-inflammatory drugs, steroids, chemotherapeutic agents (e.g.: Methotrexate) or monoclonal antibodies specific to surface molecules of T and B lymphocytes (e.g.: CD20) or cytokines (e.g.: Tumor Necrosis Factor alpha). The humanized monoclonal antibody T1h may be administered as a parenteral solution in a range of doses according to the body weight of the patient, with a variable frequency of administration which may comprise a daily, weekly or for a longer period administration. The therapeutic effect is evaluated by the reduction of the clinical activity of the disease, according to the standard criteria before, during and after finishing the treatment.

### 3.- Characterization of the effect on peripheral blood mononuclear cells of pharmaceutical compositions containing the humanized Monoclonal Antibody T1h.

Peripheral blood mononuclear cells from Rheumatoid Arthritis patients are incubated with an anti-CD3 or an anti-CD6 monoclonal antibody conjugated to biotin or fluorescent substances (e.g.: FITC, PE or PE-Cy5). The binding of biotinylated antibodies is detected with a Streptavidin, PE-Cy5.5 conjugate. At least 10 000 living cells are **acquired** in a Flow Cytometer FACScan. Dead cells are excluded with the Propidium Iodine staining.

### 4.- Characterization of the ability of the pharmaceutical compositions containing the humanized Monoclonal Antibody T1h to inhibit the CD6 binding to its ligand ALCAM.

The human epithelial cell line HEK-293 transfected with the human molecule CD6 is incubated with saturated concentration of the anti-human CD6 monoclonal antibody T1h or an isotype control, an anti-human CD3 antibody during 30 minutes at 4°C. Cells are washed and incubated with 0.5 µg/mL of the fusion protein rhALCAM-Fc (ALCAM is a CD6 ligand) for 30 min at 4°C. Then, samples are **stained** with an anti-human IgG FITC**-antibody** conjugated for 30 min at 4°C. Ten thousand living cells are **acquired** in a Flow Cytometer FACScan. Dead cells are excluded with the Propidium Iodine staining.

### Examples:

The humanized anti-human CD6 Monoclonal Antibody T1h was obtained from the hybridoma IOR- T1A with deposit No. ECACC 96112640, as described in EP 0 807 125 A2.

### Example 1: The humanized Monoclonal Antibody T1h induce a long-lasting therapeutic effect in Rheumatoid Arthritis patients.

The therapeutic effect of the humanized Monoclonal Antibody T1h **was evaluated or assessed** in 13 Rheumatoid Arthritis patients. Patients received a weekly dose of the humanized monoclonal antibody T1h during 6 weeks in a range of doses of 0.2, 0.4, 0.6 and 0.8 mg/Kg of body weight. The therapeutic effect was evaluated by the reduction of the clinical activity of the disease, considering the number of affected joints according to the standard criteria before, during and after finishing the treatment. Each curve represents the **mean** values of the percentage of improvement of the clinical sign or symptom per group of patient according to the administered dose.

### Example 2: The treatment with the humanized Monoclonal Antibody T1h transiently reduces the number of peripheral blood mononuclear cells from Rheumatoid Arthritis patients.

Peripheral blood mononuclear cells from Rheumatoid Arthritis patients treated with the humanized monoclonal antibody T1h were analyzed. The expression of the CD3 molecule, as a distinctive T lymphocyte marker, as well as the CD6 molecule was determined. The humanized monoclonal antibody T1h treatment induces a transient reduction of CD3+ and CD6+ lymphocytes. However, a recovery to the normal values does not influence the persistent clinical improvement of the disease. The study was performed by flow cytometry using a FACScan to analyze the samples. Each curve represents the values of individual patients in different time points.

### Example 3: The humanized Monoclonal Antibody T1h does not inhibit the CD6 binding to its ligand ALCAM.

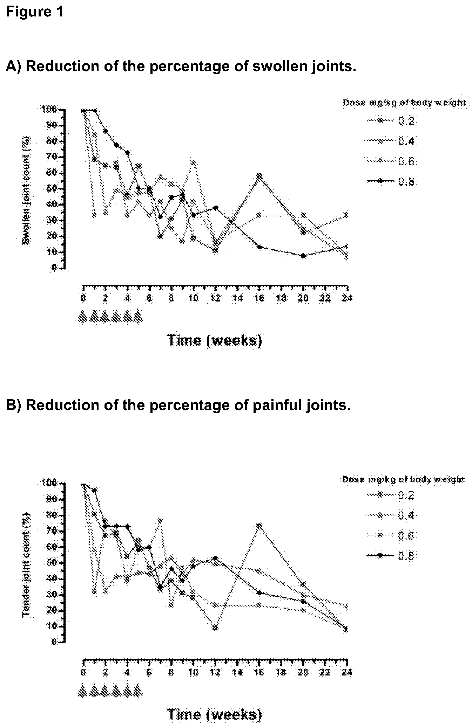

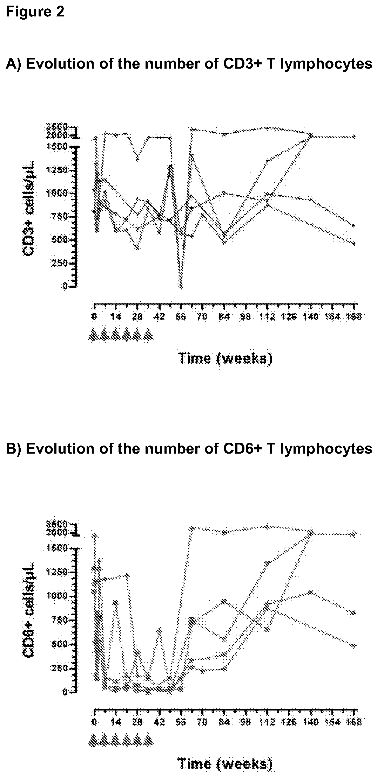

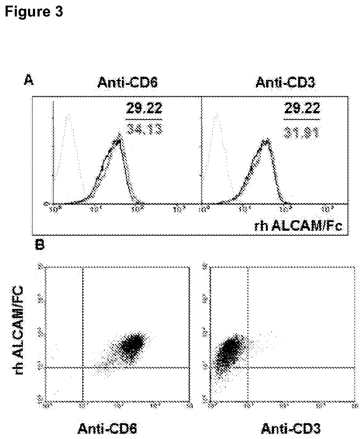

The capacity of the humanized monoclonal antibody T1h to inhibit the binding of ALCAM to the human epithelial cell line HEK-293, transfected with the human CD6 molecule was evaluated. (A) Red histogram: recognition of the human recombinant protein ALCAM bound to a human Fc fragment (rhALCAM-Fc) pre-incubated with the anti-CD6 (T1h) or anti-CD3 (control) antibodies; Black histogram: binding of the rhALCAM-Fc to non-treated cells; and Grey Histogram: labeled cells with the FITC-conjugated anti-human IgG antibody. The mean fluorescence intensity values are depicted in the figure. (B) Dot plots show the double staining for rhALCAM-Fc and anti-CD6 or anti-CD3 antibodies.

### Brief description of the figures.

**Figure 1****-**
   Therapeutic effect of the humanized monoclonal antibody T1h in Rheumatoid Arthritis patients evaluated by the reduction of the percent of swollen and tender joints.
**Figure 2****-**
   Quantification of the number of lymphocytes CD3+ and CD6+ in peripheral blood mononuclear cells from Rheumatoid Arthritis patients
**Figure 3****-**
   Demonstration of the non inhibition of the CD6 binding to the ALCAM ligand by the humanized monoclonal antibody T1 h.

## Claims

1. A pharmaceutical composition for use in the treatment of Rheumatoid Arthritis in an active phase of the disease, resistant to conventional therapies, comprising as active principle a monoclonal antibody that recognizes the human leukocyte differentiation antigen CD6, wherein the monoclonal antibody that recognizes the human leukocyte differentiation antigen CD6 does not inhibit binding of CD6 to Activated Leukocyte Adhesion Molecule (ALCAM), wherein said monoclonal antibody is a humanized antibody T1h, and wherein said humanized antibody T1h is obtained by genetic engineering methods from the secreting hybridoma TOR-T1A with deposit No. ECACC 96112640 and wherein the humanized antibody T1h comprises a heavy chain having a variable region with the following sequence: Glu Val Gln Leu Val Glu Ser Gly Gly Gly Leu Val Lys Pro Gly Gly Ser Leu Lys Leu Ser Cys Ala Ala Ser Gly Phe Lys Phe Ser Arg Tyr Ala Met Ser Trp Val Arg Gin Ala Pro Gly Lys Arg Leu Glu Trp Val Ala Thr lie Ser Ser Gly Gly Ser Tyr lie Tyr Tyr Pro Asp Ser Val Lys Gly Arg Phe Thr lie Ser Arg Asp Asn Val Lys Asn Thr Leu Tyr Leu Gin Met Ser Ser Leu Arg Ser Glu Asp Thr Ala Met Tyr Tyr Cys Ala Arg Arg Asp Tyr Asp Leu Asp Tyr Phe Asp Ser Trp Gly Gln Gly Thr Leu Val Thr Val Ser Ser; and
a light chain having a variable region with the following sequence: Asp Ile Gln Met Thr Gln Ser Pro Ser Ser Leu Ser Ala Ser Val Gly Asp Arg Val Thr Ile Thr Cys Lys Ala Ser Arg Asp Ile Arg Ser Tyr Leu Thr Trp Tyr Gln Gln Lys Pro Gly Lys Ala Pro Lys Thr Leu lie Tyr Tyr Ala Thr Ser Leu Ala Asp Gly Val Pro Ser Arg Phe Ser Gly Ser Gly Ser Gly Gin Asp Tyr Ser Leu Thr Ile Ser Ser Leu Glu Ser Asp Asp Thr Ala Thr Tyr Tyr Cys Leu Gln His Gly Glu Ser Pro Phe Thr Phe Gly Ser Gly Thr Lys Leu Glu lie Lys Arg Ala and wherein the composition is administered as a parenteral solution with a weekly frequency.

2. The pharmaceutical composition for use according to claim 1 comprising the humanized antibody T1h alone or in combination with any of the agents selected from the group consisting of:
a chemotherapeutic agent,
specific monoclonal antibodies against T and B lymphocytes surface molecules, and
a cytokine.

3. The pharmaceutical composition for use according to claim 2, wherein the monoclonal antibody against T and B lymphocytes surface molecules is an anti-CD20 antibody.

4. The pharmaceutical composition for use according to claim 2, wherein the chemotherapeutic agent is Methotrexate.

5. The pharmaceutical composition for use according to claim 2, wherein the cytokine is the Tumor Necrosis Factor alpha.

6. The pharmaceutical composition for use according to claim 1 wherein the composition is administered to a patient as injections in a range of doses from 0.05 to 1 mg/Kg of body weight.

## Patentansprüche

1. Pharmazeutische Zusammensetzung zur Verwendung bei der Behandlung von rheumatoider Arthritis in einer aktiven Phase der Krankheit, die gegen herkömmliche Therapien resistent ist, als aktives Prinzip einen monoklonalen Antikörper umfassend, der das menschliche Leukozyten-Differenzierungs-Antigen CD6 erkennt, wobei der monoklonale Antikörper, der das menschliche Leukozyten-Differenzierungs-Antigen CD6 erkennt, die Bindung von CD6 an ein aktiviertes Leukozyten-Adhäsionsmolekül (ALCAM) nicht hemmt, wobei der monoklonale Antikörper ein humanisierter Antikörper T1h ist und wobei der humanisierte Antikörper T1h durch gentechnische Verfahren aus dem sezernierenden Hybridom TOR-T1A mit der Hinterlegungsnummer ECACC 96112640 erhalten wird, und wobei der humanisierte Antikörper T1h eine schwere Kette mit einem variablen Bereich mit der folgenden Sequenz aufweist: Glu Val Gln Leu Val Glu Ser Gly Gly Gly Leu Val Lys Pro Gly Gly Ser Leu Lys Leu Ser Cys Ala Ala Ser Gly Phe Lys Phe Ser Arg Tyr Ala Met Ser Trp Val Arg Gln Ala Pro Gly Lys Arg Leu Glu Trp Val Ala Thr Ile Ser Ser Gly Gly Ser Tyr Ile Tyr Tyr Pro Asp Ser Val Lys Gly Arg Phe Thr Ile Ser Arg Asp Asn Val Lys Asn Thr Leu Tyr Leu Gln Met Ser Ser Leu Arg Ser Glu Asp Thr Ala Met Tyr Tyr Cys Ala Arg Arg Asp Tyr Asp Leu Asp Tyr Phe Asp Ser Trp Gly Gln Gly Thr Leu Val Thr Val Ser Ser; und eine leichte Kette mit einem variablen Bereich mit der folgenden Sequenz aufweist: Asp Ile Gln Met Thr Gln Ser Pro Ser Ser Leu Ser Ala Ser Val Gly Asp Arg Val Thr Ile Thr Cys Lys Ala Ser Arg Asp Ile Arg Ser Tyr Leu Thr Trp Tyr Gln Gln Lys Pro Gly Lys Ala Pro Lys Thr Leu Ile Tyr Tyr Ala Thr Ser Leu Ala Asp Gly Val Pro Ser Arg Phe Ser Gly Ser Gly Ser Gly Gln Asp Tyr Ser Leu Thr Ile Ser Ser Leu Glu Ser Asp Asp Thr Ala Thr Tyr Tyr Cys Leu Gln His Gly Glu Ser Pro Phe Thr Phe Gly Ser Gly Thr Lys Leu Glu Ile Lys Arg Ala, und wobei die Zusammensetzung als parenterale Lösung in wöchentlichem Abstand verabreicht wird.

2. Pharmazeutische Zusammensetzung zur Verwendung nach Anspruch 1, die den humanisierten Antikörper T1h alleine oder in Kombination mit einem der Mittel aufweist, die ausgewählt sind aus der Gruppe bestehend aus:
einem chemotherapeutischen Mittel,
spezifischen monoklonalen Antikörpern gegen T- und B-Lymphozyten-Oberflächenmoleküle, und
einem Zytokin.

3. Pharmazeutische Zusammensetzung zur Verwendung nach Anspruch 2, wobei der monoklonale Antikörper gegen T- und B-Lymphozyten-Oberflächenmoleküle ein Anti-CD20-Antikörper ist.

4. Pharmazeutische Zusammensetzung zur Verwendung nach Anspruch 2, wobei es sich bei dem chemotherapeutischen Mittel um Methotrexat handelt.

5. Pharmazeutische Zusammensetzung zur Verwendung nach Anspruch 2, wobei es sich bei dem Zytokin um den Tumor-Nekrosefaktor-Alpha handelt.

6. Pharmazeutische Zusammensetzung zur Verwendung nach Anspruch 1, wobei die Zusammensetzung einem Patienten in Form von Injektionen in einem Dosierungsbereich von 0,05 bis 1 mg/kg Körpergewicht verabreicht wird.

## Revendications

1. Composition pharmaceutique destinée à être utilisée dans le traitement de l'Arthrite Rhumatoïde dans une phase active de la maladie, résistante aux thérapies conventionnelles, comprenant comme principe actif un anticorps monoclonal qui reconnaît l'antigène de différenciation des leucocytes humains CD6, dans laquelle l'anticorps monoclonal qui reconnaît l'antigène de différenciation des leucocytes humains CD6 n'inhibe pas la liaison de CD6 à la Molécule d'Adhésion des Leucocytes Activée (ALCAM), dans laquelle ledit anticorps monoclonal est un anticorps humanisé T1h, et dans laquelle ledit anticorps humanisé T1h est obtenu par des méthodes de génie génétique à partir de l'hybridome sécrétant TOR-T1A au dépôt n° ECACC 96112640 et dans laquelle l'anticorps humanisé T1h comprend une chaîne lourde présentant une région variable à la séquence suivante: Glu Val Gln Leu Val Glu Ser Gly Gly Gly Leu Val Lys Pro Gly Gly Ser Leu Lys Leu Ser Cys Ala Ala Ser Gly Phe Lys Phe Ser Arg Tyr Ala Met Ser Trp Val Arg Gln Ala Pro Gly Lys Arg Leu Glu Trp Val Ala Thr Ile Ser Ser Gly Gly Ser Tyr Ile Tyr Tyr Pro Asp Ser Val Lys Gly Arg Phe Thr Ile Ser Arg Asp Asn Val Lys Asn Thr Leu Tyr Leu Gln Met Ser Ser Leu Arg Ser Glu Asp Thr Ala Met Tyr Tyr Cys Ala Arg Arg Asp Tyr Asp Leu Asp Tyr Phe Asp Ser Trp Gly Gln Gly Thr Leu Val Thr Val Ser Ser; et
une chaîne légère présentant une région variable à la séquence suivante: Asp Ile Gln Met Thr Gln Ser Pro Ser Ser Leu Ser Ala Ser Val Gly Asp Arg Val Thr Ile Thr Cys Lys Ala Ser Arg Asp Ile Arg Ser Tyr Leu Thr Trp Tyr Gln Gln Lys Pro Gly Lys Ala Pro Lys Thr Leu Ile Tyr Tyr Ala Thr Ser Leu Ala Asp Gly Val Pro Ser Arg Phe Ser Gly Ser Gly Ser Gly Gln Asp Tyr Ser Leu Thr Ile Ser Ser Leu Glu Ser Asp Asp Thr Ala Thr Tyr Tyr Cys Leu Gln His Gly Glu Ser Pro Phe Thr Phe Gly Ser Gly Thr Lys Leu Glu Ile Lys Arg Ala, et dans laquelle la composition est administrée sous forme de solution parentérale à une fréquence hebdomadaire.

2. Composition pharmaceutique destinée à être utilisée selon la revendication 1, comprenant l'anticorps humanisé T1h seul ou en combinaison avec l'un quelconque des agents choisis dans le groupe constitué de:
un agent chimiothérapeutique,
des anticorps monoclonaux spécifiques dirigés contre les molécules de surface des lymphocytes T et B, et
une cytokine.

3. Composition pharmaceutique destinée à être utilisée selon la revendication 2, dans laquelle l'anticorps monoclonal dirigé contre les molécules de surface des lymphocytes T et B est un anticorps anti-CD20.

4. Composition pharmaceutique destinée à être utilisée selon la revendication 2, dans laquelle l'agent chimiothérapeutique est le Méthotrexate.

5. Composition pharmaceutique destinée à être utilisée selon la revendication 2, dans laquelle la cytokine est le Facteur de Nécrose Tumorale alpha.

6. Composition pharmaceutique destinée à être utilisée selon la revendication 1, dans laquelle la composition est administrée à un patient sous forme d'injections dans une plage de doses de 0,05 à 1 mg/kg de poids corporel.
